# EUROPEAN PATENT APPLICATION

(11) **EP 3 718 529 A1**
(43) Date of publication of application: **07.10.2020**
(21) Application number: 19166872.2
(22) Date of filing: 02.04.2019
(51) Int. Cl.: A61K 6/00

(54) **KIT FOR TOOTH REMINERALISING AND DENTINE HYPERSENSITIVITY TREATMENT**

(71) Applicant: Koninklijke Philips N.V., 5656 AG Eindhoven (NL)
(72) Inventor: MAO, Jingliang, 5656 AE Eindhoven (NL); FENG, Jianxun, 5656 AE Eindhoven (NL)
(74) Representative: de Haan, Poul Erik

(57) **Abstract**

Provided is a kit suitable for remineralising treatment of a tooth of a subject, and in particular for treating dentine hypersensitivity. The kit comprises a first composition (120) for applying to a tooth, and a separate second composition (140) for applying to the same tooth. The first composition comprises a first cationic component including a first free or peptide-coupled amino acid having a basic side group which is protonated, and thereby cationic, when at a pH of 6-8. The first cationic component is at least partially charge-balanced by a first anionic component (130) comprising at least one of a phosphate anion, a hydrogen phosphate anion, a dihydrogen phosphate anion, a carbonate anion and a bicarbonate anion. The second composition comprises a second anionic component including a second free or peptide-coupled amino acid having an acidic side group which is deprotonated, and thereby anionic, when at a pH of 6-8. The second anionic component is at least partially charge-balanced by a second cationic component (150) comprising a calcium cation.

## Description

### FIELD OF THE INVENTION

This invention relates to a kit and method for tooth remineralising treatment, and in particular to a kit and method for treating dentine hypersensitivity. The invention further relates to the kit for use in tooth remineralising treatment, and in particular for use in treating dentine hypersensitivity.

### BACKGROUND OF THE INVENTION

Dentine hypersensitivity is characterised as a sharp short pain arising from exposed dentine, typically in response to an external stimulus such as clinical, thermal, tactile, evaporative or osmotic stimuli. It is normally experienced when the root of the tooth has been exposed to the oral environment because of gingival recession. Currently dentine hypersensitivity cannot be explained as arising from any other form of dental pathology. The conventionally accepted mechanism is the hydrodynamic theory: nerve stimulation due to movement of dentinal fluid is the cause of dentine hypersensitivity. The relationship between dentine hypersensitivity and the patency of dentine tubules has been established *in vivo*, and it has been found that occlusion of the tubules decreases that sensitivity.

The technology and materials to treat dentine hypersensitivity can be classified into two treatment categories: treatment I and treatment II. Treatment I involves desensitizing the nerves of the tooth using potassium salts, such as potassium nitrate, potassium citrate, potassium chloride, which depolarize the excited nerve fibers, thus numbing the pain. Treatment II involves occlusion of dentine tubules by plugging the tubules or coating the sensitive dentine area with a thin protective layer of material. Occlusion of open tubules blocks the above-described hydrodynamic mechanism from operating.

Treatment I is known to provide relatively short-term relief while treatment II represents a longer-term solution for dentine hypersensitivity. In practice, the product composition of treatment II may also contain materials, such as potassium salts, employed for effecting treatment I, such that the longer-term benefits of treatment II may be combined with nerve desensitization for more rapid relief of dentine hypersensitivity.

At present most dentine hypersensitivity treatment products and methods mainly operate by blocking the dentinal tubules to prevent dentinal fluid flow. Various materials have been used to occlude the open tubules, including amorphous calcium phosphate (ACP), strontium salts, such as strontium fluoride, stannous fluoride, oxalates, 2-hydroxyethylmethacrylate/glutaraldehyde, restorative resins, calcium and/or sodium phosphosilicate, arginine together with methyl vinyl ether/maleic anhydride copolymer, pyrophosphates, bioactive glass, casein phosphopeptide, Portland cement, etc. Treatments may be applied in the form of dentifrices, dentine adhesives, together with antibacterial agents, fluoride rinses, fluoride varnishes, etc. Nd-YAG laser treatment has also been used in dentine hypersensitivity treatment.

A notable example is the Pro-Argin® technology developed by Colgate®, incorporated in a toothpaste product, to treat dentine hypersensitivity. This technology works by occlusion of dentine tubules. Arginine (8%) and calcium carbonate infiltrate and block the dentinal tubules and prevent dentinal fluid flow, thus reducing dentine hypersensitivity. In application, when the desensitizing paste is applied to exposed dentine, arginine, which is positively charged under the pH conditions inside the mouth, and calcium carbonate, work together to accelerate the natural mechanisms of occlusion by binding to the negatively charged dentine surface to deposit a dentine-like mineral, which acts as a plug within the dentine tubules and a protective layer on the dentine surface.

While conventional materials and techniques have shown promise, there is a continuing desire to improve products and techniques to treat dentine hypersensitivity. In particular, it is desired to develop dentine tubule occlusion compositions having a stronger dentine affinity and which form more effective occlusion aggregates, thereby to improve treatment of dentine hypersensitivity, and related oral conditions.

### SUMMARY OF THE INVENTION

The invention is defined by the claims.

According to an aspect there is provided a kit suitable for treating dentine hypersensitivity, the kit comprising: a first composition for applying to a tooth, the first composition comprising: a first cationic component comprising a first free or peptide-coupled amino acid having a basic side group which is protonated, and thereby cationic, when at a pH of 6-8; and a first anionic component comprising at least one of a phosphate anion, a hydrogen phosphate anion, a dihydrogen phosphate anion, a carbonate anion, and a bicarbonate anion; a second composition for applying to said tooth, the second composition being separate from the first composition, wherein the second composition comprises: a second anionic component comprising a second free or peptide-coupled amino acid having an acidic side group which is deprotonated, and thereby anionic, when at a pH of 6-8; and a second cationic component comprising a calcium cation.

The first amino acid may have an α-amino group which is protonated, and thereby cationic, when at a pH of 6-8.

When the first amino acid is peptide-coupled, this may be coupled via any suitable peptide bond, such as via α-, β-, or γ-peptide bonding, providing that the basic side chain is left intact for protonation when the pH is 6-8.

The present invention is based on the realization that more effective dentine tubule occlusion may be achieved by a two-step treatment procedure using the kit defined above. The kit is suitable for treating dentine hypersensitivity but, more generally, may be employed in tooth remineralisation treatment due to its efficacy in delivering calcium cations, phosphate, hydrogen phosphate, dihydrogen phosphate, carbonate and/or bicarbonate anions, together with amino acids, to the dentine surface.

The dentine or tooth surface bears a partial negative charge (δ-). The first composition may be applied to the dentine surface in a first step. The first composition comprises a first cationic component which may be electrostatically attracted to the negatively charged (δ-) dentine surface. To this end, the first cationic component includes a first amino acid, provided as a free amino acid (without being peptide-coupled to other amino acid moieties) or in a peptide-coupled form (incorporated in a peptide). The first amino acid has a basic side group which is protonated and is thus positively charged under the near neutral pH conditions inside the mouth. The positively charged side group may be electrostatically attracted to the negatively charged (δ-) dentine surface.

Due to the basic side group, the isoelectric point (pI) of the first amino acid may be, for example, at least 7.45, in view of physiological pH generally varying between 7.35 and 7.45. The higher the pI, the better the positively charging /binding effect. The first amino acid may thus be positively charged in the physiological pH range of interest. For the avoidance of doubt, the term "isoelectric point" as used herein is defined as the pH at which the molecule carries no net charge.

The first cationic component of the first composition is at least partially charge-balanced by a first anionic component. The first anionic component comprises at least one of a phosphate anion, a hydrogen phosphate anion, a dihydrogen phosphate anion, a carbonate anion, and a bicarbonate anion. The phosphate anion, hydrogen phosphate anion, dihydrogen phosphate anion, carbonate anion and/or the bicarbonate anion is or are electrostatically associated with the first cationic component, and in particular with the first amino acid having the positively charged side group. The first amino acid included in the first cationic component therefore may act as a positively charged intermediary for binding the negatively charged phosphate, hydrogen phosphate, dihydrogen phosphate, carbonate and/or bicarbonate anion to the negatively charged (δ-) dentine surface.

The second composition may subsequently be applied to the area where the first composition has been applied. The second composition comprises a second anionic component which may be electrostatically attracted to the positively charged first cationic component already applied to the dentine surface. To this end, the second cationic component includes a second amino acid, provided as a free amino acid (without being peptide-coupled to other amino acid moieties) or in a peptide-coupled form (incorporated in a peptide). The second amino acid has an acidic side group which is deprotonated and is thus negatively charged under the near neutral pH conditions inside the mouth. The negatively charged side group may be electrostatically attracted to the first cationic component, including the first amino acid with the protonated side group, which is already present on the dentine surface.

When the second amino acid is peptide-coupled, this may be coupled via any suitable peptide bond, such as via α-, β-, or γ-peptide bonding, providing that the acidic side chain is left intact for deprotonation when the pH is 6-8.

Due to the acidic side group, the isoelectric point of the first amino acid may be, for example, less than or equal to 3.5. By the second amino acid having such a relatively low isoelectric point, the treatment efficiency associated with the second composition may be correspondingly improved.

In the second composition, the second anionic component is at least partially charge-balanced by a second cationic component. The second cationic component comprises a calcium cation. The calcium cation may be electrostatically associated with the second anionic component, and in particular with the second amino acid having the negatively charged side group. The second amino acid included in the second anionic component therefore provides a negatively charged intermediary for binding the positively charged calcium cation to the positively charged first cationic component which is already present on the dentine surface.

In this way, the kit may enable binding of amino acids, calcium cations, phosphate, hydrogen phosphate, dihydrogen phosphate, carbonate and/or bicarbonate anions to the dentine surface. The provision of phosphate, hydrogen phosphate, dihydrogen phosphate, carbonate and/or bicarbonate anions in the first composition and calcium cations in the second composition may result in introduction of significantly greater quantities of such ions to the dentine tubules and dentine surface than conventional solutions which at least partly rely on such ions being provided by the saliva of the subject. The present kit may therefore lead to rapid *in situ* formation of calcium phosphate and/or calcium carbonate in the dentine tubules and on the dentine surface. Due also to the presence of the first amino acid and the second amino acid, which may act as organic binders for the inorganic salts and are themselves protein building blocks, a dentine-like material may be provided as both a robust plug within the dentine tubules and a protective layer on the dentine surface. Effective and rapid occlusion of the dentine tubules, and concomitant relief from dentine hypersensitivity, may thus result from treatment using the kit.

The first anionic component may, for example, comprise phosphate anions. Calcium phosphate may have superior resistance to acidic conditions than calcium carbonate, when the former is used for occluding dentine tubules.

The first anionic component may further comprise at least one of a fluoride ion, a hydroxide anion, a chloride anion, a nitrate anion and a citrate anion. As well as providing oral treatment benefits, particularly in the case of fluoride ions being included in the first anionic component, such further anions mean that the first cationic component is not exclusively charge-balanced by the phosphate, hydrogen phosphate, dihydrogen phosphate, carbonate and/or bicarbonate anions. Such further anions may be more weakly associated with the positively charged side group of the first amino acid than the phosphate, hydrogen phosphate, dihydrogen phosphate, carbonate and/or bicarbonate anions. Accordingly, the presence of such further anions may mean that the first anionic component interferes to a lesser degree with the binding of the first amino acid with the dentine surface than the case where, for instance, the first anionic component consists exclusively of the phosphate, hydrogen phosphate, dihydrogen phosphate, carbonate and/or bicarbonate anion.

The molar charge ratio of the phosphate, hydrogen phosphate, dihydrogen phosphate, carbonate and/or bicarbonate anion to the first amino acid may be, for example, 1:10 to 9:10. Preferably the ratio of the phosphate, hydrogen phosphate and/or carbonate anion to the first amino acid is 1:2 to 7:10, when the protonated basic side group and/or protonated α-amino group (for histidine type amino acids when the pH increases close to 7.59) of the first amino acid carries a single positive charge at neutral pH.

The first free or peptide-coupled amino acid may be at least one of arginine, lysine, histidine, 2,3-diaminobutanoic acid, 2,4-diaminobutanoic acid, and 2,3-diaminopropionic acid. The amino, guanidino or imidazole groups included in the side groups of these amino acids may be protonated when in aqueous conditions at a pH of 6-8. Moreover, phosphate salts of lysine, arginine and histidine are all readily soluble in water at neutral pH.

The first free or peptide-coupled amino acid may be at least one of (S)-arginine, (S)-lysine, and (S)-histidine. For the avoidance of doubt, the "S" refers to the absolute configuration of the amino acid. The alternative accepted stereochemical labelling of these natural amino acids is "L". Such amino acids are naturally occurring, and thus the occluding material may have enhanced compatibility with natural dentine of the tooth.

The first free or peptide-coupled amino acid may be in a concentration of 6-12 wt.% of the first composition. First compositions formulated with this concentration of the first amino acid may lead to rapid and robust occlusion of the dentine tubules and effective dentine surface coating properties.

The second free or peptide-coupled amino acid may be at least one of aspartic acid and glutamic acid. The carboxylic acid groups included in the side groups of these amino acids may be deprotonated when in aqueous conditions at a pH of 6-8. Moreover, calcium salts of aspartic acid and glutamic acid are readily soluble in water at neutral pH.

The second free or peptide-coupled amino acid may be at least one of (S)-aspartic acid and (S)-glutamic acid. Such amino acids are naturally occurring, and thus the occluding material may have enhanced compatibility with the natural dentine of the tooth.

The second free or peptide-coupled amino acid may be in a concentration of 2-8 wt.% of the second composition. Second compositions formulated with this concentration of the second amino acid may lead to rapid and robust occlusion of the dentine tubules and effective dentine surface coating properties.

At least one of the first cationic component and the second cationic component may comprise a potassium cation. Potassium salts, such as potassium chloride, potassium nitrate, and potassium citrate, may thus be included in the first composition and/or the second composition. Potassium cations depolarize the excited nerve fibres and numb the pain associated with dentine hypersensitivity, thereby providing short-term relief to complement the occlusion provided by the other constituents of the respective compositions.

At least one of the first composition and the second composition may comprise particles of at least one of hydroxyapatite, calcium monohydrogen phosphate, and calcium monohydrogen phosphate dihydrate. The particles may, for example, have an average particle size of 0.05-1.0 µm, preferably around 0.05-0.1 µm.

Such inorganic fillers included, for example, with the first amino acid in the first composition may enable direct introduction of the inorganic material, e.g. calcium phosphate, to assist with rapid occlusion of the dentine tubules and coating of the dentine surface.

At least one of the first composition and the second composition may comprise at least one of a solvent, a surfactant and a flavouring agent. Including a solvent and/or a surfactant in the respective composition may enable tuning of the properties, e.g. the rheological characteristics, of the composition. Inclusion of a suitable flavouring agent may enhance the experience of the subject on whom the treatment is being performed, which may also promote compliance with a treatment regimen, e.g. a tooth remineralising regimen employing the kit, or a regimen using the kit for alleviating dentine hypersensitivity.

The first composition and the second composition may each independently be in the form of a liquid, a cream, a paste, a gel, or a powder. The respective composition may be aqueous and may have a pH of 6-8 when in the form of a liquid, a cream, a paste or a gel. In this manner, the side group of the first amino acid may be protonated and/or the side group of the second amino acid may be deprotonated in the respective liquid, cream, paste or gel prior to application onto the dentine surface.

The first composition and the second composition may be packaged in respective containers. The respective containers may be each independently a cartridge, a syringe barrel, a bottle, a pipette, or a tray.

In an embodiment, the kit comprises a dispensing apparatus configured to dispense the first composition and/or the second composition from the respective containers and onto a surface of a tooth. The dispensing apparatus may, for instance, comprise a dispenser pump.

The dispensing apparatus may, for example, comprise a metering arrangement for dispensing a metered amount of the respective composition. The more precise dosing of the respective compositions enabled by the metering arrangement may result in more effective dentine tubule occlusion and coating of the dentine surface, since the optimal mixing ratio of the first and second compositions for *in situ* calcium phosphate and/or calcium carbonate formation may be correspondingly achieved.

According to another aspect there is provided a kit as defined above for use in remineralising treatment of a tooth of a subject.

In an embodiment, there is provided a kit as defined above for use in treatment of dentine hypersensitivity.

According to a further aspect there is provided a method for remineralising a tooth of a subject, the method comprising: applying a first composition to the tooth, the first composition comprising: a first cationic component comprising a first free or peptide-coupled amino acid having a basic side group which is protonated, and thereby cationic, when at a pH of 6-8; and a first anionic component comprising at least one of a phosphate anion, a hydrogen phosphate anion, a dihydrogen phosphate anion, a carbonate anion and a bicarbonate anion; applying a second composition to where the first composition has been applied, wherein the second aqueous composition comprises: a second anionic component comprising a second free or peptide-coupled amino acid having an acidic side group which is deprotonated, and thereby anionic, when at a second pH of 6-8; and a second cationic component comprising calcium cations.

The method may be for, in particular, treating dentine hypersensitivity.

The first amino acid may have an α-amino group which is protonated, and thereby cationic, when at a pH of 6-8.

The applying of the first composition may, for instance, comprise massaging the first composition onto the dentine surface. The applying may, for example, be carried out with any suitable applicator, such as a toothbrush. During this step, the positively charged first amino acid, together with the phosphate, hydrogen phosphate, dihydrogen phosphate, carbonate and/or bicarbonate anions are applied onto the negatively charged (δ-) dentine surface, as previously described.

The applying of the first composition may be performed, for instance, for a period of 10-300 seconds, such as 60-120 seconds.

The method may further comprise, for example, a wiping step during which the dentine surface is gently wiped following application of the first composition.

Subsequently, the second composition is applied to the surface on which the first composition has already been applied, e.g. by massaging the second composition onto the area of the tooth which has previous received the first composition. The applying of the second composition may be performed, for instance, for a period of 10-300 seconds, such as 60-120 seconds. During this step, the negatively charged second amino acid and calcium cations are applied onto the pre-treated dentine surface (pre-treated with the first composition). An electrostatic binding is thus created between the first and second amino acids within the dentine tubules and on the dentine surface, together with rapid formation of calcium phosphate and/or calcium carbonate, as previously described.

The above method may be carried out, for example, once or twice per day.

### BRIEF DESCRIPTION OF THE DRAWINGS

Embodiments of the invention are described in more detail and by way of non-limiting examples with reference to the accompanying drawing, wherein:

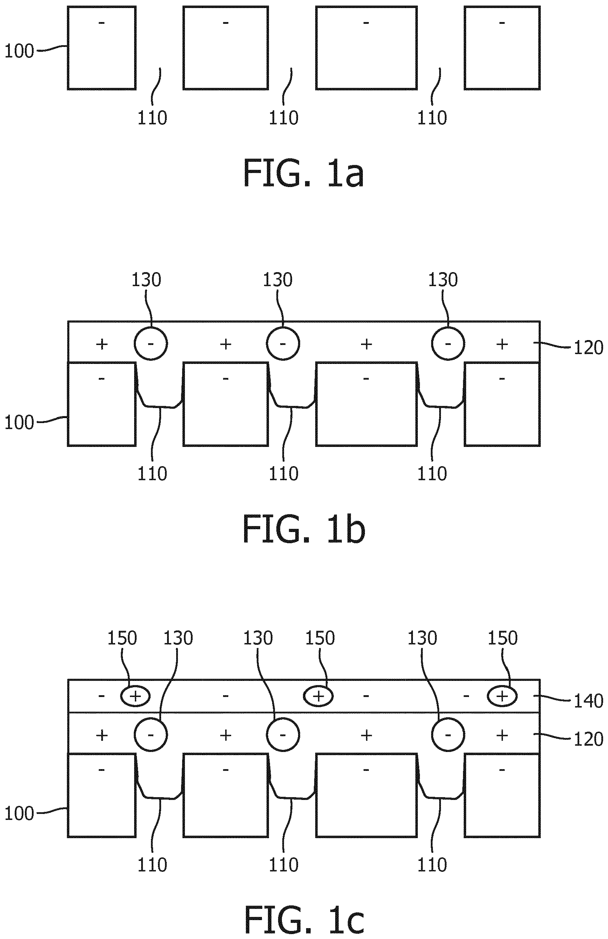

FIG. 1 schematically depicts use of the kit according to an embodiment.

### DETAILED DESCRIPTION OF THE EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a kit suitable for remineralising treatment of a tooth of a subject, and in particular for treating dentine hypersensitivity. The kit comprises a first composition for applying to a tooth, and a separate second composition for applying to the same tooth. The first composition comprises a first cationic component including a first free or peptide-coupled amino acid having a basic side group, and optionally an α-amino group, which is or are protonated, and thereby cationic, when at a pH of 6-8. The first cationic component is at least partially charge-balanced by a first anionic component comprising at least one of a phosphate anion, a hydrogen phosphate anion, a dihydrogen phosphate anion, a carbonate anion and a bicarbonate anion. The second composition comprises a second anionic component including a second free or peptide-coupled amino acid having an acidic side group which is deprotonated, and thereby anionic, when at a pH of 6-8. The second anionic component is at least partially charge-balanced by a second cationic component comprising a calcium cation.

Conventional products and methods for treating dentine hypersensitivity suffer from one or more of the following problems.

A first key problem is that the degree of occlusion of the open dentine tubules is insufficient. This may due to the occluding material not extending into the tubules to a sufficient depth, not extending across the diameter of each tubule, or both. This may result from relatively slow diffusion or dispersion of the active occlusion ingredient(s) into and over the open dentine tubules.

For example, a conventional treatment employing an arginine bicarbonate calcium carbonate complex that plugs dentinal tubules to provide immediate relief from dentinal hypersensitivity, relies, at least in part, on recruiting of calcium and phosphate ions from saliva, although calcium cations may additionally be provided by the calcium carbonate included in the composition. Such recruitment of calcium and phosphate ions from saliva is slow, due to the relatively low concentration of such ions in saliva. Moreover, calcium carbonate has a relatively low solubility in water, which means that the release, i.e. dissociation, of calcium cations is correspondingly slow. This, combined with the fact that dentine microtubules are around 5 µm in diameter, means that the conventional treatment may take a disadvantageously long time to provide effective plugging of the tubules with calcium phosphate compounds, particularly if the treatment is carried out without the help of a clinician. This is to the extent that effective plugging of the tubules may not ultimately take place.

A second key problem is the weak affinity of certain remineralisation compounds to dentine, which may lead to unstable remineralisation and poorer tubule occlusion efficiency.

For example, amorphous calcium phosphate (ACP) was originally developed in 1991 for remineralisation treatment. Amorphous calcium phosphate releases calcium cations and phosphate anions in the presence of saliva to accelerate remineralisation. The compounds formed with the dentine tubules, however, may lack stability, which leads to only a limited period of relief from dentine hypersensitivity.

A third problem, which is related to the second problem, concerns the limited duration of relief from hypersensitivity, even in spite of the treatment being provided by a dental professional. The relief effect of at least some conventional dentine hypersensitivity products may last days but not months.

Whilst it has been reported that the sensitivity relief resulting from application of a composition of arginine, bicarbonate and calcium carbonate onto sensitive teeth following scaling and root planing procedures in a dental clinic lasted for 28 days by a single treatment (Cummins D. in "Dentine hypersensitivity: from diagnosis to a breakthrough therapy for everyday sensitivity relief; J. Clin. Dent. 2009; 20(1): 1-9), thus far, no report has been found that claims a product can achieve dentine hypersensitivity relief of more than one month by one single clinical treatment.

A fourth problem relates to the inconvenience, cost and/or discomfort associated with some conventional treatments. Many products for treating dentine hypersensitivity require treatment to be performed by a clinician, such that the subject may be required to visit a dental clinic. This may be inconvenient, costly and sometimes uncomfortable from the point of view of the subject.

By using the present kit and methods, at least one of the above-described problems may be overcome. In particularly advantageous embodiments, prolonged dentine hypersensitivity relief in a natural, convenient, and cost-effective way may be achieved.

The present invention is based on the realisation that more effective dentine tubule occlusion may be achieved by a two-step treatment procedure using the kit defined above. The kit is suitable for treating dentine hypersensitivity but, more generally, may be employed in tooth remineralisation treatment due to its efficacy in delivering calcium cations, phosphate, hydrogen phosphate, dihydrogen phosphate, carbonate and/or bicarbonate anions, together with amino acids, to the dentine surface.

The dentine or tooth surface bears a partial negative charge (δ-). The first composition may be applied to the dentine surface in a first step. The first composition comprises a first cationic component which may be electrostatically attracted to the negatively charged (δ-) dentine surface. To this end, the first cationic component includes a first amino acid, provided as a free amino acid (without being peptide-coupled to other amino acid moieties) or in a peptide-coupled form (incorporated in a peptide). The first amino acid has a basic side group, and optionally an α-amino group, which is or are protonated and thus positively charged under the near neutral pH conditions inside the mouth. The positively charged side group, and α-amino group if present, may be electrostatically attracted to the negatively charged (δ-) dentine surface.

Due to the basic side group, the isoelectric point of the first amino acid may be, for example, at least 7.45, in view of physiological pH generally varying between 7.35 and 7.45. The first amino acid may thus be positively charged in the physiological pH range of interest. For the avoidance of doubt, the term "isoelectric point" as used herein is defined as the pH at which the molecule carries no net charge. Table 1 shows the isoelectric point and respective pKa values for the α-carboxyl group, α-ammonium ion, and basic side groups for the non-limiting examples of lysine, arginine and histidine.

**Table 1.**

| First Amino Acid | pKa1 (α-carboxyl group) | pKa2 (α-ammonium ion) | pKa3 (basic side group) | pI (isoelectronic point) |
|---|---|---|---|---|
| Lysine | 2.18 | 8.95 | 10.53 | 9.74 |
| Arginine | 2.17 | 9.04 | 12.48 | 10.76 |
| Histidine | 1.82 | 9.17 | 6.00 | 7.59 |

Lysine, arginine, and histidine have protonated, i.e. positively charged, basic side groups under neutral pH conditions. The basic side groups of arginine and lysine, which may be regarded as preferred first amino acids in the present context, are completely protonated at pH 6.5-8.0. Such a (first) amino acid may be included, for instance, in a concentration of 6-12 wt.% of the first composition.

More than 600 cationic peptides, i.e. comprising the peptide-coupled first amino acid, have been discovered in virtually all organisms from microbe to man. Any suitable peptide that is positively charged at neutral pH and non-toxic can be used in the first cationic component for plugging of the negatively charged (δ-) dentine tubules, and coating of the negatively charged (δ-) dentine surface. Such a peptide may be included, for instance, in a concentration of 6-12 wt.% of the first composition.

The first cationic component of the first composition is at least partially charge-balanced by a first anionic component. The first anionic component comprises a phosphate anion, a hydrogen phosphate anion, a dihydrogen phosphate anion, a carbonate anion and/or a bicarbonate anion. The phosphate anion, hydrogen phosphate anion, dihydrogen phosphate anion, carbonate anion and/or the bicarbonate anion is or are electrostatically associated with the first cationic component, and in particular with the first amino acid having the positively charged side group and, if present, α-amino group. The first amino acid included in the first cationic component therefore may act as a positively charged intermediary for binding the negatively charged phosphate anion, hydrogen phosphate anion, dihydrogen phosphate anion, carbonate anion and/or bicarbonate anion to the negatively charged (δ-) dentine surface.

Associating phosphate, hydrogen phosphate, dihydrogen phosphate, carbonate and/or bicarbonate ions with the first amino acid (by ionic bonding) may be achieved, for example, by an ion exchange reaction in solution or using a cationic ion exchange column and a relatively low concentration of phosphoric acid as the final eluent under a controlled elution rate and pH.

For example, by using a molar ratio of arginine to phosphoric acid of 6:1 in the ion exchange reaction (acid-base reaction), 50% of the guanidinium cations of arginine may be associated with the phosphate ions. For natural peptides derived from proteins, this may be done via controlled phosphoric acid hydrolysis of its raw material proteins to associate phosphate ions with the resultant peptides. Alternatively, an ion exchange reaction (acid-base reaction) between a peptide and dilute phosphoric acid under very mild reaction conditions (e.g. low temperature, and a relatively long reaction time) may be employed.

The second composition may subsequently be applied to the area where the first composition has been applied. The second composition comprises a second anionic component which may be electrostatically attracted to the positively charged first cationic component already applied to the dentine surface. To this end, the second cationic component includes a second amino acid, provided as a free amino acid (without being peptide-coupled to other amino acid moieties) or in a peptide-coupled form (incorporated in a peptide). The second amino acid has an acidic side group which is deprotonated and is thus negatively charged under the near neutral pH conditions inside the mouth. The negatively charged side group may be electrostatically attracted to the first cationic component, including the first amino acid with the protonated side group and, if present, α-amino group, which is already present on the dentine surface.

Due to the acidic side group, the isoelectric point of the first amino acid may be, for example, less than or equal to 3.5. By the second amino acid having a relatively low isoelectric point, the treatment efficiency associated with the second composition may be correspondingly improved. Table 2 shows the isoelectric point and respective pKa values for the α-carboxyl group, α-ammonium ion, and acidic side groups for the non-limiting examples of aspartic acid and glutamic acid.

**Table 2.**

| Second Amino Acid | pKa1 (α-carboxyl group) | pKa2 (α-ammonium ion) | pKa3 (acidic side group) | pI (isoelectronic point) |
|---|---|---|---|---|
| Aspartic acid | 2.09 | 9.82 | 3.86 | 2.97 |
| Glutamic acid | 2.19 | 9.67 | 4.25 | 3.22 |

Aspartic acid and glutamic acid both have a completely deprotonated, i.e. negatively charged, acidic side group under neutral pH conditions. Such an (second) amino acid may be included, for instance, in a concentration of 2-8 wt.% of the second composition.

Peptides, such as γ-polyglutamic acid (PGA), that are non-toxic and negatively charged at neutral pH, i.e. due to comprising the peptide-coupled second amino acid, may be used in the second composition. Such a peptide may be included, for instance, in a concentration of 2-8 wt.% of the second composition.

In the second composition, the second anionic component is charge-balanced by a second cationic component. The second cationic component comprises a calcium cation. The calcium cation may be electrostatically associated with the second anionic component, and in particular with the second amino acid having the negatively charged side group. The second amino acid included in the second anionic component therefore provides a negatively charged intermediary for binding the positively charged calcium cation to the positively charged first cationic component which is already present on the dentine surface.

In this way, the kit may enable binding of amino acids, calcium cations, and phosphate, hydrogen phosphate, dihydrogen phosphate, carbonate and/or bicarbonate anions to the dentine surface. The provision of phosphate, hydrogen phosphate, dihydrogen phosphate, carbonate and/or bicarbonate anions in the first composition and calcium cations in the second composition may result in introduction of significantly greater quantities of such ions to the dentine tubules and dentine surface than conventional solutions which at least partly rely on such ions being provided by the saliva of the subject. The present kit may therefore lead to rapid *in situ* formation of calcium phosphate and/or calcium carbonate in the dentine tubules and on the dentine surface. Due also to the presence of the first amino acid and the second amino acid, which may act as organic binders for the inorganic salts and are themselves protein building blocks, a dentine-like material may be provided as both a robust plug within the dentine tubules and a protective layer on the dentine surface. Effective and rapid occlusion of the dentine tubules, and concomitant relief from dentine hypersensitivity, may thus result from treatment using the kit.

Associating calcium ions with the second amino acid may be via an acid-base reaction of the second amino acid with calcium hydroxide, an acid-salt reaction between the second amino acid and calcium carbonate, or by employing an anionic ion exchange column using a low concentration of calcium hydroxide solution as the final eluent under a controlled elution rate and pH.

For example, by using a molar ratio of glutamic acid to calcium hydroxide of 2:1, 50% of the carboxylic groups of glutamic acid may be associated with calcium ions. For natural peptides derived from protein, this can be done via catalysed calcium hydroxide hydrolysis of the protein to afford peptides associated with calcium cations. Alternatively, an ion exchange reaction (acid-base reaction) between a peptide and calcium hydroxide solution under very mild reaction conditions (e.g. low temperature, and a relatively long reaction time) may be employed.

The first anionic component may, for example, comprise phosphate anions. In such an embodiment, the kit may enable relatively large amounts of calcium and phosphate ions to be provided to the dentine surface and tubules from a non-saliva source, thereby to speed up the formation of significant quantities of calcium phosphate compounds inside the dentine tubules and on the dentine surface. Calcium phosphate may be considered as the backbone of the occlusion material, while the first and second amino acids may be regarded as binders for the calcium phosphate. Moreover, calcium phosphate may have superior resistance to acidic conditions than calcium carbonate, when the former is used for occluding of dentine tubules.

Dentine is composed of approximately 20% of organic material (collagen) and 75% of inorganic material (calcium phosphate). Water mainly constitutes the remaining 5%. The collagen functions as a binder in the dentine structure. In a healthy tooth, saliva is naturally effective in reducing dentine hypersensitivity by supplying calcium cations and phosphate anions into open dentine tubules to gradually occlude them and form a surface protective layer comprising a precipitate of salivary glycoproteins and calcium phosphate. A benefit of the kit according to at least some of the herein embodiments relates to the provision of a dentine tubule occluding material which is comparable to the natural composition of the dentine, i.e. comprising calcium phosphates, proteins (or amino acids, peptides), and water.

FIG. 1 schematically depicts use of the kit according to an embodiment. In the cross-sectional view provided in pane a) of FIG. 1 the dentine surface 100 of a tooth is shown, together with dentine tubules 110 extending from the surface 100. The negative charge of the dentine surface 100 is denoted by the "-" signs.

In pane b) of FIG. 1, the first composition 120 is applied to the dentine surface 100 and into the tubules 110. The positive charge of the first cationic component of the first composition 120 is represented by the "+" signs. The first cationic component, including the protonated first amino acid, may be electrostatically attracted to the negatively charged dentine surface 100.

The first composition 120 further comprises the first anionic component 130, which includes phosphate, hydrogen phosphate, dihydrogen phosphate, carbonate and/or bicarbonate anions. The first amino acid included in the first cationic component therefore acts as a positively charged intermediary for binding the negatively charged phosphate, hydrogen phosphate, dihydrogen phosphate, carbonate and/or bicarbonate anions 130 to the negatively charged dentine surface 100, as previously described.

The applying of the first composition 120 may, for instance, comprise massaging the first composition 120 onto the dentine surface 100. The applying may, for example, be carried out with a suitable applicator provided in the kit or separately, such as a toothbrush. During this step, the positively charged first amino acid, together with the phosphate, hydrogen phosphate, dihydrogen phosphate, carbonate and/or bicarbonate anions are applied onto the negatively charged dentine surface 100, as schematically shown in FIG. 1.

The applying of the first composition 120 may be performed, for instance, for a period of 10-300 seconds, such as 60-120 seconds.

A wiping step (not shown in FIG. 1) during which the dentine surface 100 is gently wiped may be implemented following application of the first composition 120.

The first anionic component 130 may comprise phosphate anions. Calcium phosphate may have superior resistance to acidic conditions than calcium carbonate, when the former is used for occluding of dentine tubules 110.

The first anionic component 130 may further comprise at least one of a fluoride ion, a hydroxide anion, a chloride anion, a nitrate anion and a citrate anion. Sodium fluoride may, for example, be included in the first composition 120 for providing an anti-cavity function.

As well as providing oral treatment benefits, particularly in the case of fluoride ions being included in the first anionic component 130, such further anions mean that the first cationic component is not exclusively charge-balanced by the phosphate, hydrogen phosphate, dihydrogen phosphate, carbonate and/or bicarbonate anions. Such further anions may be more weakly associated with the positively charged side group of the first amino acid than the phosphate, hydrogen phosphate, dihydrogen phosphate, carbonate and/or bicarbonate anions. Accordingly, the presence of such further anions may mean that the first anionic component 130 interferes to a lesser degree with the binding of the first amino acid with the dentine surface 100 than the case where, for instance, the first anionic component 130 consists exclusively of the phosphate, hydrogen phosphate, dihydrogen phosphate, carbonate and/or bicarbonate anions.

The first free or peptide-coupled amino acid may be at least one of arginine, lysine, histidine, 2,3-diaminobutanoic acid, 2,4-diaminobutanoic acid, and 2,3-diaminopropionic acid. The amino, guanidino or imidazole groups included in the side groups of these amino acids may be protonated when in aqueous conditions at a pH of 6-8. Moreover, phosphate salts of lysine, arginine and histidine are all readily soluble in water at neutral pH.

The first free or peptide-coupled amino acid may be at least one of (S)-arginine, (S)-lysine, and (S)-histidine. Such amino acids are naturally occurring, and thus the occluding material may have enhanced compatibility with the natural dentine of the tooth.

The first free or peptide-coupled amino acid may be in a concentration of 6-12 wt.% of the first composition 120. First compositions 120 formulated with this concentration of the first amino acid may lead to rapid and robust occlusion of the dentine tubules 110 and effective dentine surface 100 coating properties.

The first cationic component may comprise a potassium cation. Potassium salts, such as potassium chloride, potassium nitrate, and potassium citrate, may thus be included in the first composition 120. Potassium cations depolarize the excited nerve fibres and numb the pain, thereby providing short-term relief to complement the occlusion provided by the other constituents of the first composition 120. By providing potassium ions in the first composition 120, the numbing effect may be advantageously provided early on, i.e. in the first step of, the treatment.

A synthetic form of hydroxyapatite created by NASA was originally shown to help restore mineral loss in the teeth of astronauts following low-gravity missions. Currently hydroxyapatite-based toothpaste is used for enamel-restoration and as an anti-cavity agent in Japan and other countries. The first composition 120 may comprise particles of at least one of hydroxyapatite, calcium monohydrogen phosphate, and calcium monohydrogen phosphate dihydrate. Preferably the filler comprises hydroxyapatite. The particles may, for example, have an average particle size of 0.05-1.0 µm, preferably around 0.05-0.1 µm.

Such fillers included, for example, with the first amino acid in the first composition 120 may enable direct introduction of the inorganic material, e.g. calcium phosphate, to assist with rapid occlusion of the dentine tubules 110 and coating of the dentine surface 100.

The combination of inert filling ingredients, such as nanoparticles of hydroxyapatite with positively charged arginine, lysine, or histidine, or peptides including such amino acids, may assist in the direct introduction of calcium phosphate to occlude the dentine tubules 110 and coat the dentine surface 100.

The first composition 120 may comprise at least one of a solvent, a surfactant and a flavouring agent. Including a solvent and/or a surfactant in the respective composition may enable tuning of the properties, e.g. the rheological characteristics, of the composition.

Flavouring agents are substances that trigger the sense of taste and/or smell. They may be used to improve the consumer perception and experience during the treatment, and thus may promote compliance with a treatment regimen, e.g. a tooth remineralising regimen employing the kit, or a regimen using the kit for alleviating dentine hypersensitivity. Mint flavours provided by, for instance, peppermint oil and spearmint oil may be used to give a sense of cleanliness and freshness. Sweeteners like xylitol or artificial sweeteners like sodium saccharin and sucralose may also, for example, be used. A preferred flavouring agent is natural peppermint oil. Any suitable flavouring agent may be employed in the first composition 120, provided that the flavouring agent is compatible with other ingredients of the first composition 120.

In a non-limiting example, if a mint oil is used as a flavouring agent, the surfactant Pluronic F-127 may also be included in the first composition 120 to solubilise the mint oil.

The first composition 120 may be, for instance, in the form of a liquid, a cream, a paste, a gel, or a powder. The first composition 120 may be aqueous and may have a pH of 6-8, preferably about pH 7, when in the form of a liquid, a cream, a paste or a gel. In this manner, the side group of the first amino acid may be protonated in the respective liquid, cream, paste or gel prior to application onto the dentine surface 100.

In pane c) of FIG. 1, the second composition 140 is applied to the pre-treated dentine surface 100 and into the tubules 110, e.g. by massaging the second composition 140 onto the area of the tooth which has previously received the first composition 120. The applying of the second composition 140 may be performed, for instance, for a period of 10-300 seconds, such as 60-120 seconds.

The treatment method schematically shown in FIG. 1 may, for example, be carried out once or twice per day.

The negative charge of the second anionic component of the second composition 140 is represented by the - signs. The second anionic component, including the deprotonated second amino acid, may be electrostatically attracted to the positively charged first cationic component. The second composition 140 further comprises the second cationic component 150, which includes calcium cations.

The second amino acid included in the second anionic component therefore acts as a negatively charged intermediary for binding the positively charged calcium cations 150 to the positively charged first cationic component which is already present on the dentine surface 100.

The second free or peptide-coupled amino acid may be at least one of aspartic acid and glutamic acid. The carboxylic acid groups included in the side groups of these amino acids may be deprotonated when under aqueous conditions at a pH of 6-8. Moreover, calcium salts of aspartic acid and glutamic acid are readily soluble in water at neutral pH.

The second free or peptide-coupled amino acid may be at least one of (S)-aspartic acid and (S)-glutamic acid. Such amino acids are naturally occurring, and thus the occluding material may have enhanced compatibility with the natural dentine of the tooth.

The second free or peptide-coupled amino acid may be in a concentration of 2-8 wt.% of the second composition 140. Second compositions 140 formulated with this concentration of the second amino acid may lead to rapid and robust occlusion of the dentine tubules 110 and effective dentine surface 100 coating properties.

The second cationic component 150 may comprise a potassium cation. Potassium salts, such as potassium chloride, potassium nitrate, and potassium citrate, may thus be included in the second composition 140, thereby to provide short-term relief to complement the occlusion provided by the other constituents of the respective compositions 120, 140.

The second composition 140 may comprise particles of at least one of hydroxyapatite, calcium monohydrogen phosphate, and calcium monohydrogen phosphate dihydrate. Preferably the filler comprises hydroxyapatite. The particles may, for example, have an average particle size of 0.05-1.0 µm, preferably around 0.05-0.1 µm.

Such fillers included, for example, with the second amino acid in the second composition 140 may enable direct introduction of the inorganic material, e.g. calcium phosphate, to assist with rapid occlusion of the dentine tubules 110 and coating of the dentine surface 100.

The second composition 140 may comprise at least one of a solvent, a surfactant and a flavouring agent. Including a solvent and/or a surfactant in the second composition 140 may enable tuning of the properties, e.g. the rheological characteristics, of the second composition 140.

Inclusion of a suitable flavouring agent may enhance the experience of the subject on whom the treatment is being performed, which may also promote compliance with a treatment regimen, e.g. a tooth remineralising regimen employing the kit, or a regimen using the kit for alleviating dentine hypersensitivity. Any suitable flavouring agent may be employed in the second composition 140, provided that the flavouring agent is compatible with other ingredients of the second composition 140. Moreover, sweeteners like xylitol, and artificial sweeteners like sodium saccharin and sucralose may, for example, be included in the second composition 140. A preferred flavouring agent is natural peppermint oil.

In a non-limiting example, if a mint oil is used as a flavouring agent, the surfactant Pluronic F-127 may also be included in the second composition 140 to solubilise the mint oil.

The second composition 140 may be in the form of a liquid, a cream, a paste, a gel, or a powder. The second composition 140 may be aqueous and may have a pH of 6-8, preferably about pH 7, when in the form of a liquid, a cream, a paste or a gel. In this manner, the side group of the second amino acid may be deprotonated in the respective liquid, cream, paste or gel prior to application onto the dentine surface 100.

The second composition 140 may be packaged in a separate container from the container used for packaging the first composition 120. The provision and storage of the respective compositions 120, 140 as two separate parts may improve the shelf-life of the compositions 120, 140 and may avoid premature reaction/binding between the ingredients of the respective compositions 120, 140 before they reach the targeted area, e.g. the interior of the dentine tubules 110. The respective containers may, for example, be each independently a cartridge, a syringe barrel, a bottle, a pipette, or a tray.

The kit may comprise a dispensing apparatus (not shown) configured to dispense the first composition 120 and/or the second composition 140 from the respective containers and onto the dentine surface 100. The dispensing apparatus may, for instance, comprise a brush. Alternatively or additionally, a pump/vacuum device may be used to withdraw contents from the respective containers, e.g. via a draw pipe, and to discharge the contents to the targeted tooth or exposed dentine surface 100 area.

The dispensing apparatus may, for example, comprise a metering arrangement for dispensing a metered amount of the respective composition 120, 140. The more precise dosing of the respective compositions enabled by the metering arrangement may result in more effective dentine tubule 110 occlusion and coating of the dentine surface 100, since the optimal mixing ratio of the first and second compositions 120, 140 for *in situ* calcium phosphate and/or calcium carbonate formation may be correspondingly achieved.

Table 3 shows a non-limiting example of a kit in which the first and second compositions 120, 140 are both in the form of a paste. Alternative kits may be readily developed by the skilled person, using the above formulation guidance.

**Table 3.**

| **First Composition** | |
|---|---|
| **Ingredient** | **% w/w in part (a)** |
| Deionised Water | 42.755 |
| Arginine | 8.000 |
| Phosphoric Acid | 1.500 |
| Potassium Nitrate | 5.000 |
| Natural Peppermint Oil | 0.600 |
| Pluronic F-127 | 2.000 |
| Hydroxyapatite Powder | 40.000 |
| Sodium Fluoride | 0.145 |

| **Second Composition** | |
|---|---|
| **Ingredient % w/w in part (b)** | |
| Deionised Water | 44.650 |
| Glutamic acid | 2.000 |
| Potassium Nitrate | 5.000 |
| Calcium Hydroxide | 1.000 |
| Glycerine | 4.000 |
| Nat. Peppermint Oil PE-5523 | 0.600 |
| Cremophor RH-40 USP | 1.500 |
| Crodateric CAB 30-LQ | 0.250 |
| Calcium Monohydrogen Phosphate Powder | 40.000 |
| Pluoronic F-127 | 1.000 |

The kit and method disclosed herein may be mainly used for dentine hypersensitivity treatment at the dental office, but may also be applicable to "take-home" or retail-type products for dentine hypersentivity treatment. Tooth cavities may also be treated using the kit, since the aggregate formed from combining the respective compositions of the kit has a strong affinity to tooth enamel. In addition, the idea to form strong occlusion binding aggregates using the oppositely charged first and second amino acids (free or peptide-coupled), together with calcium phosphate may be applied to produce artificial dentine tissue or even an artificial tooth.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A kit suitable for treating dentine hypersensitivity, the kit comprising:
a first composition for applying to a tooth, the first composition comprising:
a first cationic component comprising a first free or peptide-coupled amino acid having a basic side group which is protonated, and thereby cationic, when at a pH of 6-8; and
a first anionic component comprising at least one of a phosphate anion, a hydrogen phosphate anion, a dihydrogen phosphate anion, a carbonate anion, and a bicarbonate anion;
a second composition for applying to said tooth, the second composition being separate from the first composition, wherein the second composition comprises:
a second anionic component comprising a second free or peptide-coupled amino acid having an acidic side group which is deprotonated, and thereby anionic, when at a pH of 6-8; and
a second cationic component comprising a calcium cation.

2. The kit according to claim 1, wherein the first anionic component further comprises at least one of a fluoride ion, a hydroxide anion, a chloride anion, a nitrate anion and a citrate anion.

3. The kit according to claim 1 or claim 2, wherein the first free or peptide-coupled amino acid is at least one of arginine, lysine, histidine, 2,3-diaminobutanoic acid, 2,4-diaminobutanoic acid, and 2,3-diaminopropionic acid.

4. The kit according to any of claims 1 to 3, wherein the first free or peptide-coupled amino acid is at least one of (S)-arginine, (S)-lysine, and (S)-histidine.

5. The kit according to any of claims 1 to 4, wherein the first free or peptide-coupled amino acid is in a concentration of 6-12 wt.% of the first composition.

6. The kit according to any of claims 1 to 5, wherein the second free or peptide-coupled amino acid is at least one of aspartic acid and glutamic acid, optionally wherein the second free or peptide-coupled amino acid is at least one of (S)-aspartic acid and (S)-glutamic acid.

7. The kit according to any of claims 1 to 6, wherein the second free or peptide-coupled amino acid is in a concentration of 2-8 wt.% of the second composition.

8. The kit according to any of claims 1 to 7, wherein at least one of the first cationic component and the second cationic component comprises a potassium cation.

9. The kit according to any of claims 1 to 8, wherein at least one of the first composition and the second composition comprises particles of at least one of hydroxyapatite, calcium monohydrogen phosphate, and calcium monohydrogen phosphate dihydrate; optionally wherein the particles have an average particle size of 0.05-1.0 µm, preferably 0.05-0.1 µm.

10. The kit according to any of claims 1 to 9, wherein at least one of the first composition and the second composition comprises at least one of a solvent, a surfactant and a flavouring agent.

11. The kit according to any of claims 1 to 10, wherein the first composition and the second composition are each independently in the form of a liquid, a cream, a paste, a gel, or a powder.

12. The kit according to claim 11, wherein the respective composition is aqueous and has a pH of 6-8 when in the form of a liquid, a cream, a paste or a gel.

13. The kit according to any of claims 1 to 12, wherein the first composition and the second composition are packaged in respective containers, optionally wherein the respective containers are each independently a cartridge, a syringe barrel, a bottle, a pipette, or a tray.

14. The kit according to claim 13, comprising a dispensing apparatus configured to dispense the first composition and/or the second composition from the respective containers and onto a surface of a tooth; optionally wherein the dispensing apparatus comprises a metering arrangement for dispensing a metered amount of the respective composition.

15. A kit according to any of claims 1 to 14 for use in remineralising treatment of a tooth of a subject; optionally wherein said remineralising treatment comprises treatment of dentine hypersensitivity.

16. A method for remineralising a tooth of a subject, the method comprising:
applying a first composition to the tooth, the first composition comprising:
a first cationic component comprising a first free or peptide-coupled amino acid having a basic side group which is protonated, and thereby cationic, when at a pH of 6-8; and a first anionic component comprising at least one of a phosphate anion, a hydrogen phosphate anion, a dihydrogen phosphate anion, a carbonate anion and a bicarbonate anion; and
applying a second composition to where the first composition has been applied, wherein the second aqueous composition comprises:
a second anionic component comprising a second free or peptide-coupled amino acid having an acidic side group which is deprotonated, and thereby anionic, when at a second pH of 6-8; and a second cationic component comprising a calcium cation.
